# EUROPEAN PATENT SPECIFICATION

(11) **EP 3 986 090 B1**
(45) Date of publication and mention of the grant of the patent: **25.01.2023**
(21) Application number: 21213838.2
(22) Date of filing: 12.05.2017
(51) Int. Cl.: H05B 3/40, A24F 40/20, A24F 40/485

(54) **APPARATUS FOR HEATING SMOKABLE MATERIAL**
VORRICHTUNG ZUR ERWÄRMUNG VON RAUCHBAREM MATERIAL
APPAREIL DE CHAUFFAGE DE MATÉRIAU À FUMER

(30) Priority: 13.05.2016 US 201662336205 P
(43) Date of publication of application: 20.04.2022
(62) Divisional of application: 17726219.3
(73) Proprietor: Nicoventures Trading Limited, London WC2R 3LA (GB)
(72) Inventor: THORSEN, Mitchel, Madison, 53718 (US); MEHNERT, John, Madison, 53718 (US)
(74) Representative: Dehns

(56) References cited:
- JP-A- H03 108 472
- US-A- 6 158 530

## Description

### Technical Field

The present invention relates to apparatus arranged to heat smokable material.

### Background

Articles such as cigarettes, cigars and the like burn tobacco during use to create tobacco smoke. Attempts have been made to provide alternatives to these articles, which burn tobacco, by creating products that release compounds without burning. Examples of such products are so-called heat-not-burn products, also known as tobacco heating products or tobacco heating devices, which release compounds by heating, but not burning, the material. The material may be for example tobacco or other non-tobacco products or a combination, such as a blended mix, which may or may not contain nicotine. US6158530A relates to an apparatus for a combustible cigarette wherein a combustible cigarette is burnt by a lighter and JPH 03 108472 relates to a heatless and smokeless tobacco system.

### Summary

According to a first aspect of the present invention, there is provided an apparatus according to claim 1.

Various optional embodiments are provided by each of the dependent claims.

### Brief Description of the Drawings

Embodiments of the invention will now be described, by way of example only, with reference to the accompanying drawings, in which:
Figure 1 shows a perspective view of an example of an apparatus for heating a smokable material;
Figure 2 shows a lateral cross-sectional view of the apparatus of Figure 1 with a consumable article inserted;
Figure 3 shows a lateral cross-sectional view of the apparatus of Figure 1 without a consumable article inserted;
Figure 4 shows a perspective side view of the apparatus of Figure 1 with some external panels absent to show interior components of the apparatus;
Figure 5a shows a side view of an internal component of the apparatus of Figure 1;
Figure 5b shows a first perspective view of the internal component of the apparatus of Figure 1;
Figure 5c shows a second perspective view of the internal component of the apparatus of Figure 1;
Figure 5d shows an end view of the internal component of the apparatus of Figure 1;
Figure 6 shows an end view of an alternative internal component of the apparatus of Figure 1;
Figure 7 shows a plan view of a top panel of the apparatus for heating a smokable material;
Figure 8 shows a side view of a top panel of the apparatus of Figure 7;
Figure 9 shows a perspective view of a top panel of the apparatus of Figure 7;
Figure 10 shows a plan view of a top panel of the apparatus of Figure 7 with a consumable article inserted.

### Detailed Description

As used herein, the term "smokable material" includes materials that provide volatilised components upon heating, typically in the form of an aerosol. "Smokable material" includes any tobacco-containing material and may, for example, include one or more of tobacco, tobacco derivatives, expanded tobacco, reconstituted tobacco or tobacco substitutes. "Smokable material" also may include other, non-tobacco, products, which, depending on the product, may or may not contain nicotine. "Smokable material" may for example be in the form of a solid, a liquid, a gel or a wax or the like. "Smokable material" may for example also be a combination or a blend of materials.

Apparatus is known that heats smokable material to volatilise at least one component of the smokable material, typically to form an aerosol which can be inhaled, without burning or combusting the smokable material. Such apparatus is sometimes described as a "heat-not-burn" apparatus or a "tobacco heating product" or "tobacco heating device" or similar. Similarly, there are also so-called e-cigarette devices, which typically vaporise a smokable material in the form of a liquid, which may or may not contain nicotine. The smokable material may be in the form of or provided as part of a rod, cartridge or cassette or the like which can be inserted into the apparatus. A heater for heating and volatilising the smokable material may be provided as a "permanent" part of the apparatus or may be provided as part of the smoking article or consumable which is discarded and replaced after use. A "smoking article" in this context is a device or article or other component that includes or contains in use the smokable material, which in use is heated to volatilise the smokable material, and optionally other components.

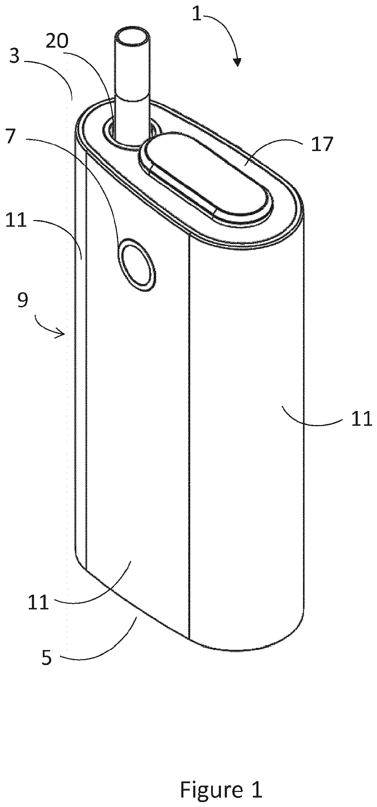

Referring initially to Figures 1 to 4, there is shown an example of an apparatus 1 arranged to heat smokable material to volatilise at least one component of said smokable material, typically to form an aerosol which can be inhaled. The apparatus 1 is a heating apparatus 1 which releases compounds by heating, but not burning, the smokable material.

A first end 3 is sometimes referred to herein as the mouth or proximal end 3 of the device 1 and a second end 5 is sometimes referred to herein as the distal end 5 of the device 1. The apparatus 1 has an on/off button 7 to allow the apparatus 1 as a whole to be switched on and off as desired by a user.

The apparatus 1 comprises a housing 9 for locating and protecting various internal components of the apparatus 1. In the example shown, the housing 9 comprises a uni-body sleeve 11 that encompasses the perimeter of the apparatus 1, capped with a top panel 17 which defines generally the 'top' of the apparatus 1 and a bottom panel 19 which defines generally the 'bottom' of the apparatus 1. In another example the housing comprises a front panel, a rear panel and a pair of opposite side panels in addition to the top panel 17 and the bottom panel 19.

The top panel 17 and/or the bottom panel 19 may be removably fixed to the uni-body sleeve 11, to permit easy access to the interior of the apparatus 1, or may be "permanently" fixed to the uni-body sleeve 11, for example to deter a user from accessing the interior of the apparatus 1. In an example, the panels 17 and 19 are made of a plastics material, including for example glass-filled nylon formed by injection moulding, and the uni-body sleeve 11 is made of aluminium, though other materials and other manufacturing processes may be used.

The top panel 17 of the apparatus 1 has an opening 20 at the mouth end 3 of the apparatus 1 through which, in use, a consumable article 21 containing smokable material may be inserted into the apparatus 1 and removed from the apparatus 1 by a user.

The housing 9 has located or fixed therein a heater arrangement 23, control circuitry 25 and a power source 27. In this example, the heater arrangement 23, the control circuitry 25 and the power source 27 are laterally adjacent (that is, adjacent when viewed from an end), with the control circuitry 25 being located generally between the heater arrangement 23 and the power source 27, though other locations are possible.

The control circuitry 25 may include a controller, such as a microprocessor arrangement, configured and arranged to control the heating of the smokable material in the consumable article 21 as discussed further below.

The power source 27 may be for example a battery, which may be a rechargeable battery or a non-rechargeable battery. Examples of suitable batteries include for example a lithium-ion battery, a nickel battery (such as a nickel-cadmium battery), an alkaline battery and/ or the like. The battery 27 is electrically coupled to the heater arrangement 23 to supply electrical power when required and under control of the control circuitry 25 to heat the smokable material in the consumable (as discussed, to volatilise the smokable material without causing the smokable material to burn).

An advantage of locating the power source 27 laterally adjacent to the heater arrangement 23 is that a physically large power source 25 may be used without causing the apparatus 1 as a whole to be unduly lengthy. As will be understood, in general a physically large power source 25 has a higher capacity (that is, the total electrical energy that can be supplied, often measured in Amp-hours or the like) and thus the battery life for the apparatus 1 can be longer.

In one example, the heater arrangement 23 is generally in the form of a hollow cylindrical tube, having a hollow interior heating chamber 29 into which the consumable article 21 comprising the smokable material is inserted for heating in use. Different arrangements for the heater arrangement 23 are possible. For example, the heater arrangement 23 may comprise a single heating element or may be formed of plural heating elements aligned along the longitudinal axis of the heater arrangement 23. The or each heating element may be annular or tubular, or at least part-annular or part-tubular around its circumference. In an example, the or each heating element may be a thin film heater. In another example, the or each heating element may be made of a ceramics material. Examples of suitable ceramics materials include alumina and aluminium nitride and silicon nitride ceramics, which may be laminated and sintered. Other heating arrangements are possible, including for example inductive heating, infrared heater elements, which heat by emitting infrared radiation, or resistive heating elements formed by for example a resistive electrical winding.

In one particular example, the heater arrangement 23 is supported by a stainless steel support tube and comprises a polyimide heating element. The heater arrangement 23 is dimensioned so that substantially the whole of the smokable material when the consumable article 21 is inserted in the apparatus 1 so that substantially the whole of the smokable material is heated in use.

The or each heating element may be arranged so that selected zones of the smokable material can be independently heated, for example in turn (over time) or together (simultaneously) as desired.

The heater arrangement 23 in this example is surrounded along at least part of its length by a thermal insulator 31. The insulator 31 helps to reduce heat passing from the heater arrangement 23 to the exterior of the apparatus 1. This helps to keep down the power requirements for the heater arrangement 23 as it reduces heat losses generally. The insulator 31 also helps to keep the exterior of the apparatus 1 cool during operation of the heater arrangement 23. In one example, the insulator 31 may be a double-walled sleeve which provides a low pressure region between the two walls of the sleeve. That is, the insulator 31 may be for example a "vacuum" tube, i.e. a tube that has been at least partially evacuated so as to minimise heat transfer by conduction and/or convection. Other arrangements for the insulator 31 are possible, including using heat insulating materials, including for example a suitable foam-type material, in addition to or instead of a double-walled sleeve.

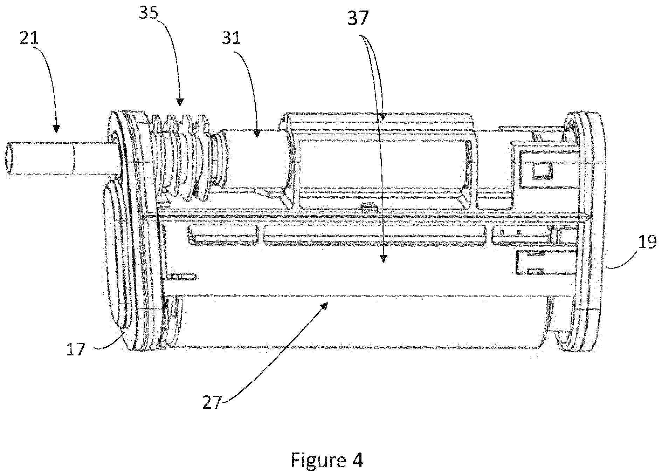

The housing 9 may further comprises various internal support structures 37 (best seen in Figure 4) for supporting all internal components, as well as the heating arrangement 23.

The apparatus 1 further comprises a collar 33 which extends around and projects from the opening 20 into the interior of the housing 9 and a generally tubular chamber 35 which is located between the collar 33 and one end of the vacuum sleeve 31.

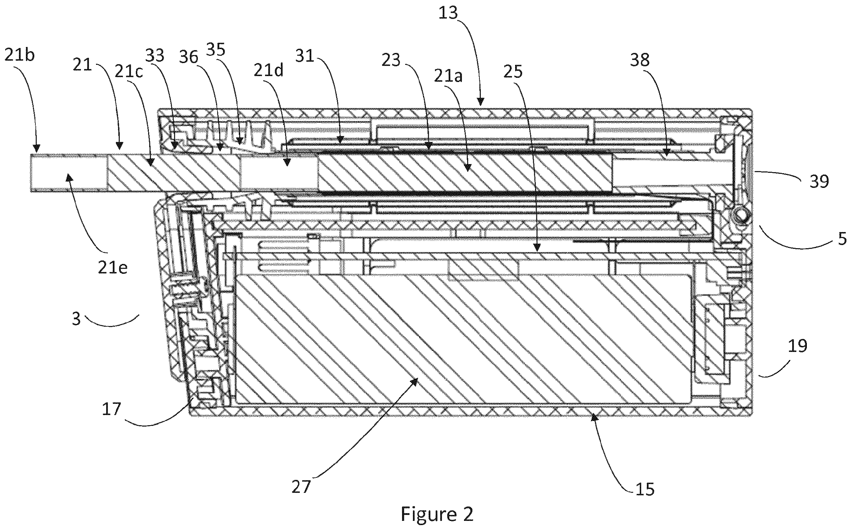

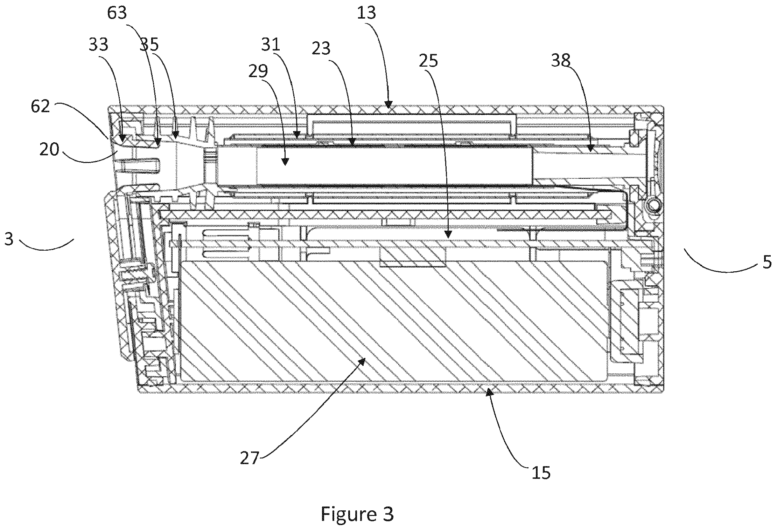

One end of the chamber 35 connects to and is supported by the collar 33 and the other end of the chamber 35 connects to the one end of the vacuum sleeve 31 and hence supports the vacuum sleeve 31. Accordingly, as best seen in Figure 3, the collar 33, the chamber 35 and the vacuum tube 31/heater arrangement 23 are arranged co-axially, so that, as best seen in Figure 2, when the consumable 21 is inserted in the apparatus 1, it extends through the collar 33 and the chamber 35 into the heater chamber 29.

As mentioned above, in this example, the heater arrangement 23 is generally in the form of a hollow cylindrical tube and this tube is in fluid communication with the opening 20 at the mouth end 3 of the device 1 via the chamber 35 and the collar 33.

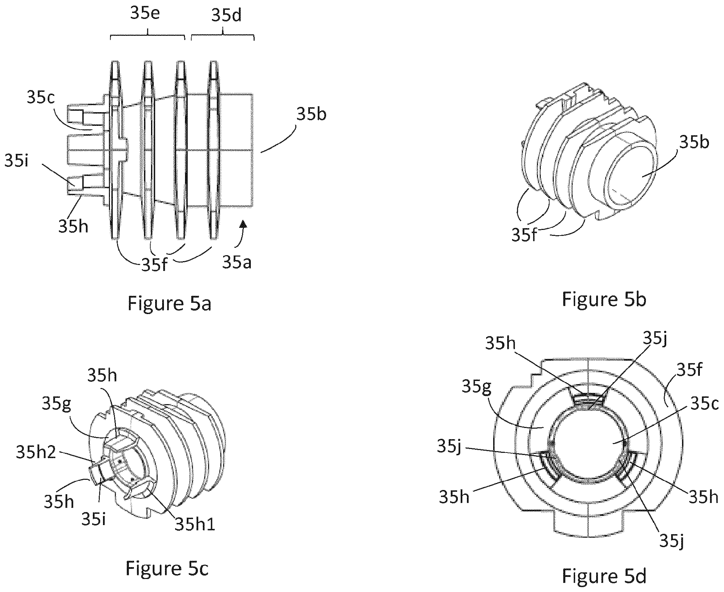

Referring now to Figures 5a to 5d, in this example, the chamber 35 comprises a tubular body 35a that has a first open end 35b and a second open end 35c. The tubular body 35a comprises a first section 35d that extends from the first open end 35b to approximately half away along the tubular body 35a and a second section 35e that extends from approximately half away along the tubular body 35a to the second open end 35c. The first section 35d has a substantially constant internal diameter and the second section 35e has an internal diameter that tapers towards the second open end 35c.

The chamber 35 further comprises a cooling structure 35f, which in this example, comprises a plurality of cooling fins 35f spaced apart along the body 35a, each of which is arranged circumferentially around the body 35a.

The chamber 35 also comprises a flange portion 35g around the second open end 35c and a plurality of projections or clips 35h also arranged around the second open end 35c. Each clip 35h is generally 'L' shaped and comprises a first portion 35h1 that is joined to the flange portion 35g and a second portion 35h2 that is generally perpendicular to first portion 35h1 and which extends in a direction generally parallel to the longitudinal axis of the tubular body 35a. Each second portion 35h2 comprises a stepped surface 35i that faces towards an axis that extends along the longitudinal axis of the tubular body 35a and which stepped surface 35i is slightly curved.

As best seen in Figure 3, in this example, the chamber 35 is located in the housing 9 between the collar 33 and the vacuum tube 31/heater 23. More specifically, (i) at the second end 35c, the flange 35g butts an end portion of a polyimide tube of the heater arrangement 23 with the clips 35h resiliently engaging with the polyimide tube via their stepped surfaces 35i and the outer surfaces of the clips mating with an inside of the vacuum sleeve 31 (ii) at the first open end 35b, the chamber 35 connects to the collar 33 by means of ridges 60, which form part of the collar 33 and project into the chamber 35. The ridges 60 are angled from a first end 62 of the collar 33 to a second end 63 of the collar towards an axis that extends along the longitudinal axis of the collar 33 and chamber 35. The ridges lie flush with the internal surface of the chamber 35 to form a snug fit.

As is best appreciated from Figure 2, the inner diameter of the first section 35d of the hollow chamber 35 is larger than the external diameter of the consumable article 2. There is therefore an air gap 36 between the hollow chamber 35 and the consumable article 2 when it is inserted in the apparatus over at least part of the length of the hollow chamber 35. The air gap 36 is around all of the circumference of the consumable article 21 in that region.

As best seen in Figure 5c and Figure 5d, at the second open end 35c, the chamber 35 comprises a plurality (in this example 3) of small lobes or ridges 35j arranged circumferentially around an inner surface of the chamber 35 at the periphery of second open end 35c. Each of the lobes 35j extends a small distance in a direction parallel to the longitudinal axis of the chamber 35 and also extends a small amount radially at the second open end 35c. Together, the lobes 35j provide a gripping section that grips the consumable article 21 in order to correctly position and retain the portion of the consumable article 21 that is within the chamber 35 when the consumable article 21 is within the apparatus 1. Between them, the lobes 35j gently compress or pinch the consumable article 21 in the region or regions of the consumable article that are contacted by the lobes 35j. The lobes 35j may be comprised of a resilient material (or be resilient is some other way) so that they deform slightly (for example compress) to better grip the consumable article 21 when it is inserted in the apparatus 1 but then regain their original shape when the consumable article 21 is removed from the apparatus 1. The lobes 35j may be formed integrally with the chamber 35 or may be separate components that are attached within the chamber 35. The inner diameter around the lobes, may be, for example, 5.377mm.

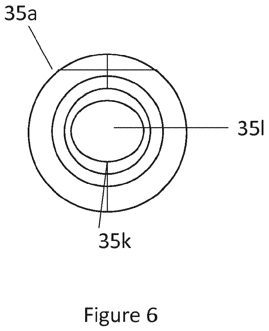

In an alternative example shown in Figure 6, a resilient gripping section 35k within the hollow chamber 35 defines a substantially oval aperture 351 which, may extend along the longitudinal axis of the hollow chamber 35, and which when the consumable article 21 is inserted in the apparatus 1, gently compresses or pinches the section of the consumable article 21 that is in the oval apertures 351 so that this section of the consumable article 21 is deformed from being circular to being oval in cross section. In one example, the gripping section 35k is located towards the first open end 35b. In one example, the width of the oval section could be increased or decreased to increase or decrease the insertion/retention force. In a further example, small grooves (not shown) could be added in the surface of the oval aperture 351 that would interfere with the consumable article 21 rather than the entire surface area of the oval aperture 35l. This would minimize insertion/removal sensitivity to the transitions of the various consumable article components (tobacco, tipping paper, paper tube) passing through the gripping section 35k.

In a further example a combination of the lobes 35j and the oval gripping section 35k could be used to retain the consumable article 21 in the hollow chamber 35. For example, an oval gripping section 35k and the arrangement of lobes 35j could be spaced apart longitudinally in the hollow chamber 35 and act separately to retain an inserted consumable article 21 in place, or, the lobes 35j could be arranged around the surface of the oval gripping section 35k.

The chamber 35 may be formed of for example a plastics material, including for example polyether ether ketone (PEEK).

Referring again to Figures 2 to 4, in an example, the heating chamber 29 has a region 38 of reduced internal diameter towards the distal end 5. This region 37 provides an end stop for the consumable article 21 passed through the opening at the mouth end 3. This region 38 of reduced internal diameter, may for example, be provided by a hollow tube of the type described in detail in our co-pending application US provisional patent application no. 62/185,227, filed on June 26, 2015.

The apparatus 1 may further comprise a door 39 at the distal end 5 that opens and closes an opening in the rear panel to provide access to the heating chamber 29 so that the heating chamber can be cleaned. Examples of suitable doors are also discussed in more detail in our co-pending application 62/185,227.

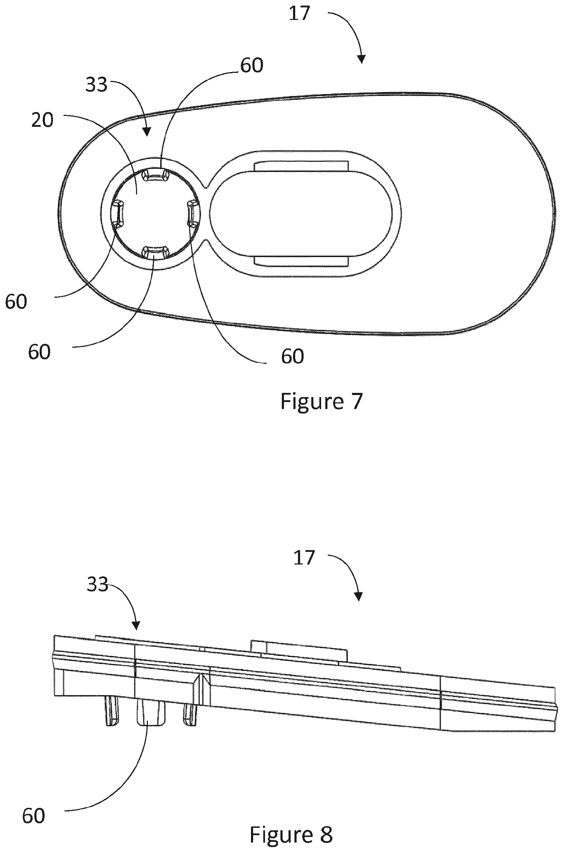

Referring now to Figures 7 to 10 in particular, there is shown an example of the top panel 17 of the apparatus 1. The top panel 17 generally forms the front end 3 of the housing 9 of the apparatus. The top panel 17 supports the collar 33 which defines an insertion point in the form of the opening 20 through which the consumable article 21 is removably inserted into the apparatus 1 in use.

The collar 33 extends around and projects from the opening 20 into the interior of the housing 9. In one example, the collar 33 is integral with the top panel 17 of the housing so the collar 33 and the top panel 17 form a single piece. In an alternative example, the collar 33 is a distinct element from the top panel 17, but can be attached to the top panel 17 through an attachment, such as a locking mechanism, adhesive, screws. Other attachments that are suitable for attaching the collar 33 to the top panel 17 may be used.

In this example, the collar 33 comprises a plurality of ridges 60 arranged circumferentially around the periphery of the opening 20 and which project into the opening 20. The ridges 60 take up space within the opening 20 such that the open span of the opening 20 at the locations of the ridges 60 is less than the open span of the opening 20 at the locations without the ridges 60. The ridges 60 are configured to engage with a consumable article 21 inserted into the apparatus to assist in securing it within the apparatus 1.

In one example, the ridges 60 are circumferentially equally spaced around the periphery of the opening 20. In one example, there are four ridges 60, in other examples there may be more or fewer than four ridges 60.

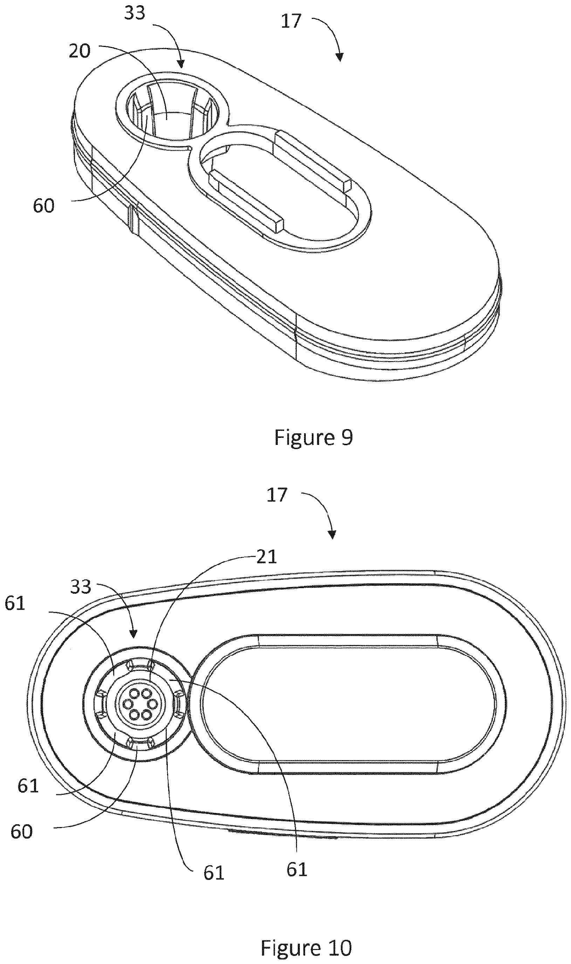

Figure 9 shows a plan view of the top panel 17 of the apparatus with a consumable article 21 inserted into the opening 20. The ridges 60 project into the opening 20 to engage with the consumable article 21. The open spaces 61 defined by adjacent pairs of ridges 60 and the consumable article 21 form ventilation paths 61 around the exterior of the consumable article 21. These ventilation paths 61, as will be explained in more detail below, allow hot vapours that have escaped from the consumable article 21 to exit the apparatus 1 and allow cooling air to flow into the apparatus 1 around the consumable 21. The example in Figure 10 shows four ventilation paths 61 located around the periphery of the consumable article 21, which provide ventilation for the apparatus 1 although there may be more or less such ventilation paths 61.

As mentioned above, the ridges 60 project radially into the opening 20 but, as best appreciated from Figure 8, they also extend from the top panel 17 into the housing 9. The projection of the ridges 60 are angled towards each other, such that as the ridges 60 extend into the housing, the distance between the ridges 60 decreases. As best seen in Figure 3, the projection of the ridges 60 into the housing enables the collar 35 to connect to the chamber 35 by means of the ridges 60 extending through the first open end 35b of the chamber 35 and engaging an inner wall of the chamber 35.

Referring again particularly to Figure 2, in one example, the consumable article 21 is in the form of a cylindrical rod which has or contains smokable material 21a at a rear end in a section of the consumable article 21 that is within the heating arrangement 23 when the consumable article 21 is inserted in the apparatus 1. A front end of the consumable article 21 extends from the apparatus 1 and acts as a mouthpiece assembly 21b which includes one or more of a filter for filtering aerosol and/or a cooling element 21c for cooling aerosol. The filter/cooling element 21c is spaced from the smokable material 21a by a space 21d and is also spaced from the tip of mouthpiece assembly 21b by a further space 21e. The consumable article 21 is circumferentially wrapped in an outer layer (not shown). In one example, the outer layer of the consumable article 21 is permeable to allow some heated volatilised components from the smokable material to escape the consumable article 21.

In operation, the heater arrangement 23 will heat the consumable article 21 to volatilise at least one component of the smokable material 21a.

The primary flow path for the heated volatilised components from the smokable material 21a is axially through the consumable article 21, through the space 21d, the filter/cooling element 21c and the further space 21e before entering a user's mouth through the open end of the mouthpiece assembly 21b. However, some of the volatilised components may escape from the consumable article 21 through its permeable outer wrapper and into the space 36 surrounding the consumable article 21 in the chamber 35.

It would be undesirable for the volatilised components that flow from the consumable article 21 into the chamber 35 to be inhaled by the user, because these components would not pass through the filter/cooling element 21c and thus be unfiltered and not cooled.

Advantageously, the volume of air surrounding the consumable article 21 in the chamber 35 and the fin-cooled interior wall of the chamber 35 causes at least some of the volatilised components that escape the consumable article 21 through its outer layer to cool and condense on the interior wall of the chamber 35 preventing those volatilised components from being possibly inhaled by a user.

This cooling effect may be assisted by cool air that is able to enter from outside the apparatus 1 into the space 36 surrounding the consumable article 21 in the chamber 35 via the ventilation paths 61, which allows fluid to flow into and out of the apparatus. A ventilation path 61 will be defined between a pair of the plurality of neighbouring ridges 60 to provide ventilation around the outside of the consumable article 21 at the insertion point.

In one example, a second ventilation path 61 is provided between a second pair of neighbouring ridges for at least one heated volatilised components to flow from the consumable article 21 at a second location. Therefore ventilation is provided around the outside of the consumable article 21 at the insertion point by the first and second ventilation paths 61.

Furthermore, heated volatilised components that escape the consumable article 21 through its outer wrapper do not condense on the internal wall of the chamber 35 and are able to flow safely out of the apparatus 1 via the ventilation paths 61 without being inhaled by a user.

The chamber 35 and the ventilation both aid in reducing the temperature and the content of water vapour composition released in heated volatilised components from the smokable material.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein. In addition, this disclosure may include other inventions not presently claimed, but which may be claimed in future.

## Claims

1. An apparatus arranged to heat but not burn smokable material to volatilise at least one component of the smokable material, the apparatus comprising:
a housing (9) having an insertion point formed in a collar (33) at one end, through which a consumable article (21) comprising smokable material can be removably inserted into the apparatus in use;
at least one heater arrangement (23) arranged within the housing (9) for heating said smokable material within the consumable article (21) when in use; and
a chamber (35) comprising a tubular body (35a), the chamber (35) located in the housing (9) between the collar (33) and the at least one heater arrangement (23);
wherein, when the consumable article (21) is inserted into the apparatus, the housing (9) comprises ventilation around the outside of said consumable article (21) at the insertion point to enable at least one heated volatilised component from said smokable material to exit the apparatus and/or air to enter the apparatus;
wherein the collar (33) comprises a plurality of ridges (60) arranged circumferentially around the insertion point that project into the insertion point;
wherein a first ventilation path (61) is defined between a first neighbouring pair of the plurality of ridges (60) and wherein the ventilation is provided by the first ventilation path (61);
wherein a second ventilation path (61) is defined between a second neighbouring pair of the plurality of ridges (60) and wherein the ventilation is additionally provided by the second ventilation path (61);
wherein the plurality of ridges (60) protrude into the insertion point to reduce the span of the insertion point at the location of the plurality of ridges (60);
wherein the collar (33) is distinct from the housing (9) and an open end of the chamber (35) connects to and is supported by the collar (33);
wherein the chamber (35) comprises a plurality of small lobes or ridges (35j) arranged circumferentially around an inner surface of the chamber (35); and
wherein apparatus further comprises a heater chamber (29) arranged co-axially with the chamber (35) and the collar (33) such that, when the consumable article (21) is inserted in the apparatus, it extends through the collar (33) and the chamber (35) into the heater chamber (29);
and wherein the apparatus further comprises:
control circuitry (25) configured and arranged to control the heating of the smokable material in the consumable article (21); and
a power source (27) comprising a battery (27), wherein the battery (27) is electrically coupled to the heater arrangement (23) to supply electrical power when required and under control of the control circuitry (25) to heat the smokable material in the consumable article (21) to volatise the smokable material without causing the smokable material to burn.

2. Apparatus as claimed in claim 1, wherein the ventilation is configured to enable the at least one heated volatilised component that has entered a chamber (35), surrounding said consumable article (21), to exit the apparatus.

3. Apparatus as claimed in any preceding claim, wherein the collar comprises four ridges.

4. Apparatus as claimed in any preceding claim, wherein the plurality of ridges are configured to engage with said consumable article received within the apparatus in use.

5. Apparatus as claimed in any preceding claim, wherein the heater arrangement is an inductive heating arrangement.

6. Apparatus as claimed in any preceding claim, wherein the heater arrangement is surrounded along at least part of its length by a thermal insulator.

## Patentansprüche

1. Einrichtung, die so eingerichtet ist, dass sie rauchbares Material erhitzt, aber nicht verbrennt, um mindestens einen Bestandteil des rauchbaren Materials zu verflüchtigen, wobei die Einrichtung umfasst:
ein Gehäuse (9), das einen Einsatzpunkt aufweist, der in einem Kragen (33) an einem Ende gebildet ist, durch den ein Verbrauchsartikel (21), der rauchbares Material umfasst, im Gebrauch in die Einrichtung abnehmbar eingesetzt werden kann;
mindestens eine Heizanordnung (23), die innerhalb des Gehäuses (9) eingerichtet ist, um das rauchbare Material im Gebrauch innerhalb des Verbrauchsartikels (21) zu erhitzen; und
eine Kammer (35), die einen röhrenförmigen Körper (35a) umfasst, wobei sich die Kammer (35) im Gehäuse (9) zwischen dem Kragen (33) und der mindestens einen Heizanordnung (23) befindet;
wobei, wenn der Verbrauchsartikel (21) in die Einrichtung eingesetzt ist, das Gehäuse (9) Belüftung um die Außenseite des Verbrauchsartikels (21) am Einsatzpunkt umfasst, um zu ermöglichen, dass mindestens ein erhitzter verflüchtigter Bestandteil aus dem rauchbaren Material aus der Einrichtung austritt und/oder Luft in die Einrichtung eintritt;
wobei der Kragen (33) eine Vielzahl von umfänglich um den Einsatzpunkt angeordneten Rippen (60) umfasst, die in den Einsatzpunkt vorragen;
wobei ein erster Belüftungsweg (61) zwischen einem ersten benachbarten Paar der Vielzahl von Rippen (60) definiert ist und wobei die Belüftung durch den ersten Belüftungsweg (61) bereitgestellt wird;
wobei ein zweiter Belüftungsweg (61) zwischen einem zweiten benachbarten Paar der Vielzahl von Rippen (60) definiert ist und wobei die Belüftung zusätzlich durch den zweiten Belüftungsweg (61) bereitgestellt wird;
wobei die Vielzahl von Rippen (60) in den Einsatzpunkt vorragt, um die Öffnung des Einsatzpunkts an der Stelle der Vielzahl von Rippen (60) zu verringern;
wobei der Kragen (33) separat vom Gehäuse (9) ist und ein offenes Ende der Kammer (35) mit dem Kragen (33) verbunden ist und von diesem gestützt wird;
wobei die Kammer (35) eine Vielzahl von kleinen Lappen oder Rippen (35j) umfasst, die umfänglich um eine Innenfläche der Kammer (35) eingerichtet ist; und
wobei die Einrichtung weiter eine Heizkammer (29) umfasst, die derart koaxial mit der Kammer (35) und dem Kragen (33) eingerichtet ist, dass sie sich durch den Kragen (33) und die Kammer (35) in die Heizkammer (29) erstreckt, wenn der Verbrauchsartikel (21) in der Einrichtung eingesetzt ist;
und wobei die Einrichtung weiter umfasst:
Steuerschalttechnik (25), die konfiguriert und eingerichtet ist, um das Heizen des rauchbaren Materials im Verbrauchsartikel (21) zu steuern; und
eine Leistungsquelle (27), die eine Batterie (27) umfasst, wobei die Batterie (27) elektrisch mit der Heizanordnung (23) gekoppelt ist, um bei Bedarf elektrische Leistung zuzuführen, und unter Kontrolle der Steuerschaltung (25) steht, um das rauchbare Material im Verbrauchsartikel (21) so zu erhitzen, dass sich das rauchbare Material verflüchtigt, ohne zu bewirken, dass das rauchbare Material brennt.

2. Einrichtung nach Anspruch 1, wobei die Belüftung konfiguriert ist, um zu ermöglichen, dass der mindestens eine erhitzte verflüchtigte Bestandteil, der in eine Kammer (35) eingetreten ist, die den Verbrauchsartikel (21) umgibt, aus der Einrichtung austritt.

3. Einrichtung nach einem vorstehenden Anspruch, wobei der Kragen vier Rippen umfasst.

4. Einrichtung nach einem vorstehenden Anspruch, wobei die Vielzahl von Rippen konfiguriert ist, um im Gebrauch in den Verbrauchsartikel einzugreifen, der innerhalb der Einrichtung aufgenommen ist.

5. Einrichtung nach einem vorstehenden Anspruch, wobei die Heizanordnung eine induktive Heizanordnung ist.

6. Einrichtung nach einem vorstehenden Anspruch, wobei die Heizanordnung entlang zumindest eines Teils ihrer Länge von einem Wärmeisolator umgeben ist.

## Revendications

1. Appareil agencé pour chauffer mais ne pas brûler une matière pouvant être fumée pour volatiliser au moins un composant de la matière pouvant être fumée, l'appareil comprenant :
un boîtier (9) présentant un point d'insertion formé dans un collier (33) à une extrémité, à travers lequel un article consommable (21) comprenant une matière pouvant être fumée peut être inséré de manière amovible dans l'appareil en utilisation ;
au moins un agencement de dispositif de chauffage (23) agencé dans le boîtier (9) pour chauffer ladite matière pouvant être fumée dans l'article consommable (21) lors de l'utilisation ; et
une chambre (35) comprenant un corps tubulaire (35a), la chambre (35) étant placée dans le boîtier (9) entre le collier (33) et le au moins un agencement de dispositif de chauffage (23) ;
dans lequel, lorsque l'article consommable (21) est inséré dans l'appareil, le boîtier (9) comprend une ventilation autour de l'extérieur dudit article consommable (21) au point d'insertion pour permettre à au moins un composant volatilisé chauffé à partir de ladite matière pouvant être fumée de sortir de l'appareil et/ou à de l'air d'entrer dans l'appareil ;
dans lequel le collier (33) comprend une pluralité d'arêtes (60) agencées de manière circonférentielle autour du point d'insertion qui se projettent dans le point d'insertion ;
dans lequel un premier trajet de ventilation (61) est défini entre une première paire voisine de la pluralité d'arêtes (60) et dans lequel la ventilation est fournie par le premier trajet de ventilation (61) ;
dans lequel un second trajet de ventilation (61) est défini entre une seconde paire voisine de la pluralité d'arêtes (60) et dans lequel la ventilation est par ailleurs fournie par le second trajet de ventilation (61) ;
dans lequel la pluralité d'arêtes (60) font saillie dans le point d'insertion pour réduire l'étendue du point d'insertion à l'emplacement de la pluralité d'arêtes (60) ;
dans lequel le collier (33) est distinct du boîtier (9) et une extrémité ouverte de la chambre (35) est reliée au collier (33) et supportée par celui-ci ;
dans lequel la chambre (35) comprend une pluralité de petits lobes ou arêtes (35j) agencés de manière circonférentielle autour d'une surface intérieure de la chambre (35) ; et
dans lequel un appareil comprend en outre une chambre de dispositif de chauffage (29) agencée de manière coaxiale avec la chambre (35) et le collier (33) de telle sorte que, lorsque l'article consommable (21) est inséré dans l'appareil, il s'étend à travers le collier (33) et la chambre (35) dans la chambre de dispositif de chauffage (29) ;
et dans lequel l'appareil comprend en outre :
un ensemble de circuits de commande (25) configuré et agencé pour commander le chauffage de la matière pouvant être fumée dans l'article consommable (21) ; et
une source d'énergie (27) comprenant une batterie (27), dans lequel la batterie (27) est couplée électriquement à l'agencement de dispositif de chauffage (23) pour fournir de l'énergie électrique lorsque cela est nécessaire et sous la commande de l'ensemble de circuits de commande (25) pour chauffer la matière pouvant être fumée dans l'article consommable (21) pour volatiliser la matière pouvant être fumée sans amener la matière pouvant être fumée à brûler.

2. Appareil selon la revendication 1, dans lequel la ventilation est configurée pour permettre au au moins un composant volatilisé chauffé qui est entré dans une chambre (35), entourant ledit article consommable (21), de sortir de l'appareil.

3. Appareil selon une quelconque revendication précédente, dans lequel le collier comprend quatre arêtes.

4. Appareil selon une quelconque revendication précédente, dans lequel la pluralité d'arêtes sont configurées pour venir en prise avec ledit article consommable reçu dans l'appareil en utilisation.

5. Appareil selon une quelconque revendication précédente, dans lequel l'agencement de dispositif de chauffage est un agencement de chauffage par induction.

6. Appareil selon une quelconque revendication précédente, dans lequel l'agencement de dispositif de chauffage est entouré le long d'au moins une partie de sa longueur d'un isolant thermique.
